## Europäisches Patentamt
## European Patent Office
## Office européen des brevets

(19)

(11) Veröffentlichungsnummer: **0 046 876**
**B1**

(12) **EUROPÄISCHE PATENTSCHRIFT**

(45) Veröffentlichungstag der Patentschrift:
30.11.83

(51) Int. Cl.³: **C 07 C 7/08**, B 01 D 3/42 //
C10G7/08, C10G7/12

(21) Anmeldenummer: 81105820.5

(22) Anmeldetag: 23.07.81

(54) **Verfahren zur Aufarbeitung des Sumpfproduktes von Extraktivdestillationsprozessen zur Gewinnung reiner Kohlenwasserstoffe.**

(30) Priorität: 30.08.80 DE 3032780

(43) Veröffentlichungstag der Anmeldung:
10.03.82 Patentblatt 82/10

(45) Bekanntmachung des Hinweises auf die Patenterteilung:
30.11.83 Patentblatt 83/48

(84) Benannte Vertragsstaaten:
AT BE DE FR GB IT NL

(56) Entgegenhaltungen:
DE - A - 2 103 858
DE - A - 2 823 901
US - A - 2 593 931
US - A - 2 655 462
US - A - 3 464 895
US - A - 3 496 070

(73) Patentinhaber: Krupp-Koppers GmbH, Moltkestrasse 29,
D-4300 Essen 1 (DE)

(72) Erfinder: Preusser, Gerhard, Dr., Dipl.-Chem.,
Lönsberg 24, D-4300 Essen 1 (DE)
Erfinder: Schulze, Martin, Krüdenscheider Weg 83,
D-5604 Neviges (DE)

Verfahren zur Aufarbeitung des Sumpfproduktes
von Extraktivdestillationsprozessen zur Gewinnung reiner Kohlenwasserstoffe

Die Erfindung betrifft ein Verfahren zur Aufarbeitung des Sumpfproduktes von Extraktivdestillationsprozessen zur Gewinnung reiner Kohlenwasserstoffe, bei dem das aufzuarbeitende Sumpfprodukt der Extraktivdestillationskolonne in den mittleren Teil einer mit Böden und einem Sumpfkocher versehenen Abtriebskolonne eingeleitet wird, in der die zu gewinnenden Kohlenwasserstoffe über Kopf abdestilliert werden, während das Lösungsmittel aus dem Sumpf der Abtriebskolonne abgezogen und gegebenenfalls nach dem indirekten Wärmeaustausch mit anderen Produktströmen zur Lösungsmittelaufgabe der Extraktivdestillationskolonne zurückgeführt wird.

Die Extraktivdestillation ist heute ein in der Praxis weit verbreitetes Verfahren zur Trennung von Stoffgemischen, insbesondere von Kohlenwasserstoffgemischen, die sich auf Grund der Siedelage ihrer Komponenten rein destillativ nicht oder nur in unbefriedigendem Umfange trennen lassen. Als Beispiele hierfür seien die Gewinnung von Reinaromaten (Benzol, Toluol, Xylol) aus aromatenhaltigen Einsatzprodukten, die Trennung von Mono- und Diolefinen sowie die Abtrennung dieser Olefine von nichtaromatischen Kohlenwasserstoffen genannt.

Für die Durchführung derartiger Extraktivdestillationsverfahren sind bereits eine Vielzahl unterschiedlicher selektiver Lösungsmittel vorgeschlagen worden, wobei sich N-substituierte Morpholine, insbesondere das N-Formylmorpholin, besonders bewährt haben. Die Trennwirkung des verwendeten selektiven Lösungsmittels beruht dabei darauf, daß durch seine Gegenwart die Dampfdrücke der einzelnen Komponenten des zu trennenden Stoffgemisches in der Weise verändert werden, daß die Dampfdruckunterschiede zwischen den Komponenten, die sich im Extrakt anreichern sollen, und jenen Komponenten, die sich im Raffinat anreichern sollen, vergrößert werden. Dadurch können die letzteren als leichter siedende Fraktion über Kopf aus der Extraktivdestillationskolonne abdestilliert werden, während die schwerer siedenden Komponenten zusammen mit dem überwiegenden Teil des Lösungsmittels als Sumpfprodukt in der Extraktivdestillationskolonne (sogen. Extraktphase) anfallen. Um die als Extrakt zu gewinnenden Komponenten vom Lösungsmittel abzutrennen, ist deshalb die eingangs beschriebene Aufarbeitung des Sumpfproduktes der Extraktivdestillationskolonne in einer nachgeschalteten Abtriebskoloone erforderlich. Zur Erreichung eines guten Trenneffektes wird hierbei üblicherweise die Abtriebskolonne mit einem Rückfluß aus den über Kopf der Kolonne gehenden Komponenten des Extraktes betrieben. Das aus dem Sumpf der Abtriebskolonne abfließende Lösungsmittel weist eine erhöhte Temperatur auf, die beispielsweise bei der Aromatengewinnung in Abhängigkeit von den Betriebsbedingungen der Abtriebskolonne (Druck und Temperatur) im Bereich zwischen 150 und 240°C liegt. Vor der Wiedereinleitung des aus dem Sumpf der Abtriebskolonne abfließenden Lösungsmittels in die Extraktivdestillationskolonne ist es aber erforderlich, dasselbe auf ca. 90 bis 120°C abzukühlen.

Um den Wärmeinhalt des aus der Abtriebskolonne abgezogenen heißen Lösungsmittels auszunutzen, ist deshalb bereits vorgeschlagen worden, das Lösungsmittel vor der Wiedereinleitung in die Extraktivdestillationskolonne im indirekten Wärmeaustausch mit anderen Produktströmen zu kühlen. So wird beispielsweise in der DE-A-2 823 901 ein Verfahren zur Butadienabtrennung durch Extraktivdestillation beschrieben, bei dem die Butadien-Schlußbehandlungskolonne im Wärmeaustausch mit dem dampfförmigen Lösungsmittelstrom aus der Abtriebskolonne beheizt wird. Aus der US-A-2 593 931 ist ein Extraktionsverfahren zur Trennung von Aromaten und Nichtaromaten bekannt, das unter Verwendung von Phenol als Lösungsmittel arbeitet. Hierbei erfolgt die Aufarbeitung der Extraktphase in einem zweistufigen Verfahren, wobei der Wärmeinhalt des abgetriebenen Phenols zur Beziehung der Abtriebskolonne sowie zur Produktvorwärmung zwischen den beiden Verfahrensstufen genutzt wird. Weitere im Recherchenbericht genannte Entgegenhaltungen betreffen andere Methoden zur Verbesserung des Betriebes der Abtriebskolonne und damit der Lösungsmittelwiedergewinnung. So beschreibt die US-A-3 496 070 ein Extraktivdestillationsverfahren zur Gewinnung von C$_4$- und C$_5$-Kohlenwasserstoffen, bei dem ein Teilstrom der flüssigen Extraktphase dem dampfförmigen Kopfprodukt der Abtriebskolonne zugesetzt wird. Dadurch soll der Druck in der Abtriebskolonne sowie dem nachgeschalteten Kondensator herabgesetzt werden. Die US-A-2 655 462 betrifft wiederum ein Extraktionsverfahren, das unter Verwendung von Phenol als Lösungsmittel arbeitet und der Raffination von Schmierölen dient. Hierbei sollen sich teilweise stabile Phenol-Öl-Komplexe bilden, weshalb der Abtrieb des Phenols in einer besonderen Kolonne erfolgt. Dabei wird ein Teilstrom aus dem Oberteil der Abtriebskolonne abgezogen und nach einer thermischen Behandlung, die der Zersetzung des Phenol-Öl-Komplexes dient, wieder in die Abtriebskolonne zurückgeführt. Die US-A-3 464 895 beschreibt ein Verfahren zur Kontrolle und Optimierung der Wärmezufuhr zu einer normalen Fraktionierkolonne. Diese Entgegenhaltung hat daher keinerlei Relevanz zum erfindungsgemäßen Verfahren. Entsprechendes gilt auch für die DE-A-2 103 858, die eine Regeleinrichtung für eine Fraktionierkolonne betrifft.

In einer von der Anmelderin herausgegebenen Werbeschrift (Koppers-Bericht 333 b v. IX. 69) wird schließlich ein Verfahrensschema zur Gewinnung von reinem o-Xylol aus Reformat durch Extraktivdestillation beschrieben, bei dem das aus der Abtriebskolonne ablaufende Lösungsmittel zunächst im indirekten Wärmeaustausch zur Aufheizung des Sumpfproduktes der Abtriebskolonne und der Extraktivdestillationskolonne mit herangezogen wird. Durch eine derartige Arbeitsweise wird jedoch das Lösungsmittel nicht in dem Umfang abgekühlt, daß seine Wiedereinhaltung in die Extraktivdestillationskolonne ohne weiteres möglich ist. Es muß deshalb vor der Wiedereinleitung in die Extraktivdestillationskolonne zunächst noch eine weitere Abkühlung in einem Luftkühler erfahren, wodurch natürlich ein Teil seines Wärmeinhaltes ungenützt verloren geht.

Tatsächlich stellen die Wirtschaftlichkeit der Lösungsmittelwiedergewinnung aus dem Sumpfprodukt der Extraktivdestillation und die damit verbundene Nutzung des Wärmeinhaltes des von den Kohlenwasserstoffen des Extraktes abgetrennten Lösungsmittels Faktoren dar, die die Wirtschaftlichkeit des Gesamtverfahrens entscheidend beeinflussen.

Der Erfindung lag deshalb die Aufgabe zugrunde, die Aufarbeitung des Sumpfproduktes von Extraktivdestillationsprozessen zur Gewinnung reiner Kohlenwasserstoffe durch eine verbesserte Nutzung des Wärmeinhaltes des dabei abgetrennten Lösungsmittels sowie eine allgemeine Optimierung des Abtriebsprozesses wirtschaftlicher zu gestalten. Die vorgegebene Zielsetzung soll dabei gleichzeitig durch eine verbesserte Regelung der Fahrweise der Abtriebskolonne unterstützt werden.

Das der Lösung dieser Aufgabe dienende Verfahren ist erfindungsgemäß dadurch gekennzeichnet, daß aus der Abtriebskolonne oberhalb des Aufgabebodens für das Sumpfprodukt der Extraktivdestillationskolonne über einen sogen. Rückflußverdampfungsboden mit erhöhtem Flüssigkeitsspiegel ein Seitenstrom abgezogen wird, der im indirekten Wärmeaustausch mit dem zur Extraktivdestillationskolonne zurückfließenden Lösungsmittel aufgeheizt und danach in die Abtriebskolonne auf den Rückflußverdampfungsboden oder einen der darüber liegenden Böden wieder eingeleitet wird, wobei die Lösungsmittelkonzentration auf dem Rückflußverdampfungsboden durch die Rückflußmenge gesteuert und auf einen Wert zwischen 10 und 70 Gew.-% gehalten wird.

Bei der erfindungsgemäßen Arbeitsweise wird also ein erheblicher Teil der für den Betrieb der Abtriebskolonne benötigten Wärmemenge, die normalerweise im Sumpfkocher dieser Kolonne durch kostenintensiven Mitteldruckdampf zur Verfügung gestellt werden muß, durch den indirekten Wärmeaustausch mit dem von den Kohlenwasserstoffen des Extraktes abgetrennten Lösungsmittel zur Verfügung gestellt. Im Hinblick auf die Temperaturverhältnisse in der Abtriebskolonne, deren Betriebstemperatur ja von unten nach oben abnimmt, ist es jedoch nicht sinnvoll, diesen Wärmeaustausch im Unterteil der Abtriebskolonne bei hohem Temperaturniveau vorzunehmen. Die erfindungsgemäße Arbeitsweise sieht deshalb vor, daß der Seitenstrom, der im indirekten Wärmeaustausch mit dem zur Extraktivdestillationskolonne zurückfließenden Lösungsmittel aufgeheizt werden soll, erst aus dem Oberteil der Abtriebskolonne, das heißt oberhalb des Aufgabebodens, abgezogen wird. Zu diesem Zweck ist an der dafür vorgesehenen Stelle im Oberteil der Abtriebskolonne ein sogen. Rückflußverdampfungsboden angeordnet, der sich von den übrigen Kolonnenböden durch einen erhöhten Flüssigkeitsspiegel unterscheidet. Dies kann ohne Schwierigkeiten durch die Anwendung einer geeigneten Bodenkonstruktion, z. B. eines sogen. Kaminbodens, herbeigeführt werden. Der Rückflußverdampfungsboden befindet sich dabei normalerweise 3 bis 5 Böden oberhalb des Aufgabebodens. Der vom Rückflußverdampfungsboden abgezogene Seitenstrom wird nach seiner Wiederaufheizung, die im indirekten Wärmeaustausch mit dem Lösungsmittel in einem entsprechend angeordneten Kocher erfolgt, wieder in die Abtriebskolonne zurückgeleitet. Die Wiedereinleitung erfolgt dabei vorzugsweise auf den Rückflußverdampfungsboden selbst oder gegebenenfalls 1 bis 5 Böden oberhalb desselben. Das Lösungsmittel wird gleichzeitig beim Passieren des Kochers abgekühlt, wobei die Betriebsbedingungen im Kocher vorzugsweise so einreguliert werden, daß die Abkühlung des Lösungsmittels bis zu der Temperatur erfolgt, mit der es ohne jede weitere Zwischenkühlung wieder auf die Extraktivdestillationskolonne aufgegeben werden kann.

Bei der Durchführung des erfindungsgemäßen Verfahrens ist allerdings zu berücksichtigen, daß die Wiederaufheizung des Seitenstromes im indirekten Wärmeaustausch mit dem Lösungsmittel nicht dazu führen darf, daß dabei der gesamte auf die Abtriebskolonne aufgegebene Rückfluß verdampft, weil ein Teil des Rückflusses mit den Lösungsmittelresten aus dem Rückflußverdampfungsboden in den Unterteil der Abtriebskolonne ablaufen muß. Zur Einhaltung optimaler Bedingungen ist deshalb vorgesehen, daß die Lösungsmittelkonzentration auf dem Rückflußverdampfungsboden durch die auf die Abtriebskolonne aufgegebene Rückflußmenge gesteuert und einem Wert zwischen 10 und 70 Gew.-% Lösungsmittel, vorzugsweise zwischen 20 und 50 Gew.-%, gehalten wird.

Das heißt, die auf die Abtriebskolonne aufgegebene Rückflußmenge richtet sich nach der Lösungsmittelkonzentration, die auf dem Rückflußverdampfungsboden ermittelt wurde. Wenn die Lösungsmittelkonzentration zu stark ansteigt, muß die Rückflußmenge entsprechend vergrößert werden, während umgekehrt bei einem Absinken der Lösungsmittelkonzentration die Rückflußmenge verringert werden kann. Zur Regelung des erfindungsgemäßen Verfahrens ist es deshalb erforderlich, die Lösungsmittelkonzentration auf dem Rückflußverdampfungsboden in geeigneter Weise zu bestimmen und den so erhaltenen Wert als Regelgröße für die Rückflußmenge in der weiter oben beschriebenen Art und Weise zu verwenden.

Für die Ermittlung der Lösungsmittelkonzentration stehen dabei prinzipiell folgende bekannte Meßmethoden zur Verfügung:

1. Gaschromatographische Bestimmung,
2. Dichtemessung,
3. Messung des Berechnungsindexes,
4. Temperaturmessung.

Die Messungen unter 1. bis 3. erfordern allerdings aufwendige Meßgeräte mit Probeleitungen vom Rückflußverdampfungsboden zum Meßgerät und zurück. Bei der Temperaturmessung gemäß 4. ist der Meßaufwand zwar relativ gering. Da die Abtriebskolonne in der Regel jedoch bei Unterdruck betrieben wird, gehen Druckschwankungen, die verschiedene Ursachen haben können, in die Messung ein und verfälschen den Meßwert.

In dem Bestreben, die vorstehend geschilderten Nachteile der bekannten Meßverfahren — die natürlich prinzipiell zur Anwendung beim erfindungsgemäßen Verfahren geeignet sind — zu vermeiden, wurde nun überraschenderweise gefunden, daß die Messung der Temperaturdifferenz zwischen dem Rückflußverdampfungsboden und einem darüber liegenden Boden einen von allen auf die Abtriebskolonne einwirkenden Störfaktoren unabhängigen Meßwert liefert, der als Regelgröße für die Rückflußmenge dienen kann. Vorzugsweise wird dabei die Temperatur auf dem Rückflußverdampfungsboden und einem 4 bis 6 Böden höher liegenden Boden ermittelt, wobei die resultierende Temperaturdifferenz die Rückflußmenge in dem Sinne regelt, daß bei steigendem $\Delta t$-Wert die Rückflußmenge vergrößert und bei fallendem $\Delta t$-Wert verkleinert wird. Es hat sich gezeigt, daß bei Anwendung der erfindungsgemäßen Temperaturdifferenzmessung, die auf die Abtriebskolonne aufgegebene Rückflußmenge zuverlässig und völlig unabhängig von etwa in der Abtriebskolonne auftretenden Druckschwankungen gesteuert werden kann, wobei der apparative Aufwand zur Durchführung des erfindungsgemäßen Verfahrens verhältnismäßig gering ist.

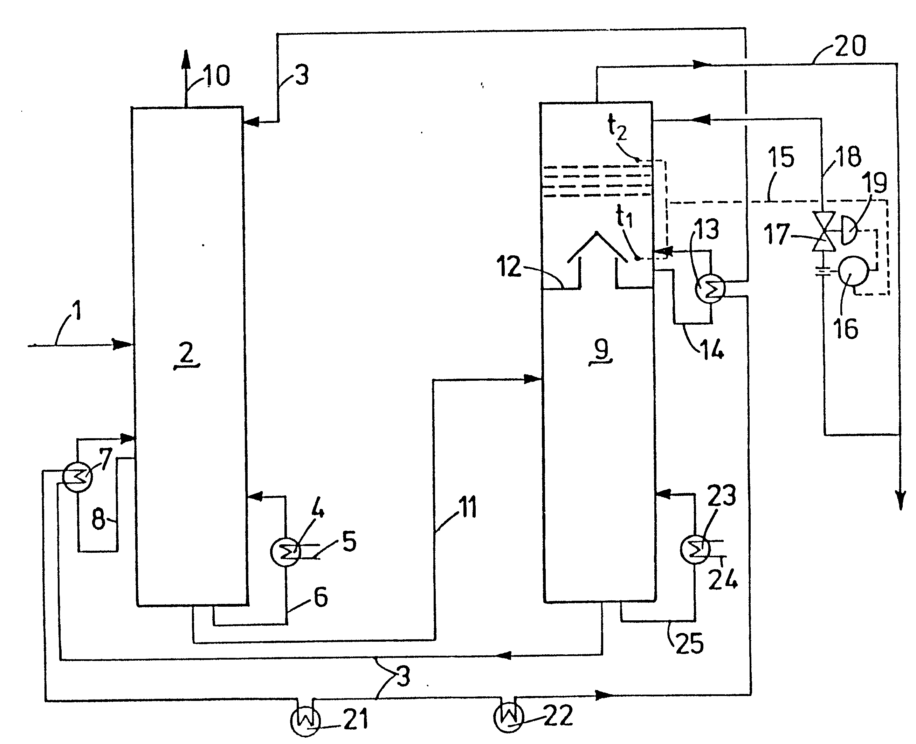

Weitere Einzelheiten des erfindungsgemäßen Verfahrens sowie dessen Verknüpfung mit der vorgeschalteten Extraktivdestillation sollen nachfolgend an Hand des in der Abbildung dargestellten Fließschemas erläutert werden, welches gleichzeitig ein Ausführungsbeispiel betrifft.

In dem Fließ-Schema sind selbstverständlich nur die zur Erläuterung des Verfahrensablaufes unerläßlichen Anlagenteile dargestellt, während sonstige Nebeneinrichtungen wie Pumpen, Ventile usw. nicht aufgeführt wurden.

Das aufzuarbeitende Einsatzprodukt, das aus dem Benzol gewonnen werden soll, wird über die Leitung 1 in den mittleren Teil der mit Böden versehenen Extraktivdestillationskolonne 2 eingeleitet. Das Einsatzprodukt in Leitung 1 weist dabei eine Temperatur von 80°C auf und hat folgende Zusammensetzung:

| | |
|---|---|
| Nichtaromaten | 33 Gew.-% |
| Benzol | 67 Gew.-% |

Als selektives Lösungsmittel wird im vorliegenden Falle N-Formylmorpholin verwendet, wobei das Lösungsmittel mit einer Temperatur von 103°C über die Leitung 3 auf den Kopf der Extraktivdestillationskolonne 2 aufgegeben wird. Für die Beheizung der Extraktivdestillationskolonne 2 ist am Kolonnensumpf der Kocher 4 vorgesehen, in dem eine indirekte Beheizung des Sumpfproduktes mit Mitteldruckdampf erfolgt, der durch die Leitung 5 fließt. Das aufzuheizende Sumpfprodukt der Extraktivdestillationskolonne 2 wird über die Leitung 6 abgezogen und gelangt nach Passieren des Kochers 4 auf einen der Böden oberhalb des Sumpfes in die Extraktivdestillationskolonne 2 zurück. Zusätzlich ist am Unterteil der Extraktivdestillationskolonne 2 noch ein weiterer Kocher 7 inhalliert, in dem das über die Leitung 8 fließende Produkt im indirekten Wärmeaustausch mit dem durch die Leitung 3 fließenden, von der Abtriebskolonne 9 kommenden Lösungsmittel aufgeheizt wird.

Die leichter siedenden Komponenten des Einsatzproduktes verlassen die Extraktivdestillationskolonne 2 über Kopf durch die Leitung 10, während das anfallende Sumpfprodukt über die Leitung 11 angezogen wird. Das Produkt in Leitung 10 (Raffinatphase) hat dabei folgende Zusammensetzung:

| | |
|---|---|
| Nichtaromaten | 97 Gew.-% |
| Benzol | 2 Gew.-% |
| Lösungsmittel | 1 Gew.-% |

Das Sumpfprodukt in Leitung 11 (Extraktphase) enthält das im Lösungsmittel gelöste Benzol. Zur weiteren Aufarbeitung wird dieses Sumpfprodukt der Extraktivdestillation über die Leitung 11 in den mittleren Teil der mit Böden versehenen Abtriebskolonne 9 eingeleitet. In dieser Kolonne, die bei einem Druck von 0,7 – 0,5 bar betrieben wird, ist der 3. Boden oberhalb des Aufgabebodens als Kaminboden mit erhöhtem Flüssigkeitsspiegel ausgebildet und dient erfindungsgemäß als sogen. Rückflußverdampfungsboden 12. In Höhe dieses Bodens ist an der Abtriebskolonne 9 der Kocher 13 angeordnet, der im indirekten Wärmeaustausch mit dem durch die Leitung 3 fließenden Lösungsmittel beheizt wird. Vom Rückflußverdampfungsboden 12 wird dabei über die Leitung 14 ein Seitenstrom abgezogen und zur Wiederaufheizung durch den Kocher 13 geleitet und anschließend auf den Rückflußverdampfungsboden 12 zurückgegeben. Die im Kocher 13 zur Wiederaufheizung des Seitenstromes zur Verfügung gestellte Wärmemenge richtet sich natürlich nach dem Wärmeinhalt des Lösungsmittels in diesem Teil der Leitung 3 sowie nach der Endtemperatur, bis zu der das Lösungsmittel beim Passieren des Kochers 13 abgekühlt werden soll.

Im vorliegenden Falle soll das Lösungsmittel, was die bevorzugte Arbeitsweise ist, bis auf die Temperatur angekühlt werden, mit der es wieder auf die Extraktivdestillationskolonne 2 aufgegeben werden kann. Gleichzeitig soll die Lösungsmittelkonzentration auf den Rückflußverdampfungsboden 12 im Bereich zwischen 20 und 50 Gew.-% gehalten werden. Die zu diesem Zweck notwendige Regelung der Rückflußmenge erfolgt hier über die weiter oben beschriebene Temperaturdifferenzmessung. Daher ist auf dem Rückflußverdampfungsboden 12 der Temperaturmeßpunkt $t_1$ und auf dem 4. Boden darüber der Temperaturmeßpunkt $t_2$ vorgesehen. Die ersten vier über dem Rückflußverdampfungsboden 12 liegenden Böden sind in der Abbildung durch unterbrochene Linien dargestellt. Die zwischen $t_1$ und $t_2$ ermittelte Temperaturdifferenz $\Delta t$ wird über die unterbrochen gezeichnete Impulsleitung 15 auf den Durchflußmengenregler 16 übertragen, der über die Stellung des Ventiles 17 die Menge des in der Leitung 18 fließenden Rückflusses regelt, der über diese Leitung auf den Kopf der Abtriebskolonne 9 aufgegeben wird. Der Stellantrieb 19 dient dabei zur Betätigung des Ventiles 17. Im vorliegenden Falle muß der ermittelte $\Delta t$-Wert im Bereich zwischen 3 und 12°C liegen. Wird dieser Bereich nach oben überschritten, so wird durch eine entsprechende Betätigung des Ventiles 17 die Zufuhr von Rückfluß über die Leitung 18 erhöht, während im umgekehrten Falle, wenn der angegebene Bereich für den $\Delta t$-Wert nicht erreicht wird, eine Verminderung der Rückflußmenge dadurch herbeigeführt wird, daß das Ventil 17 automatisch weiter geschlossen wird. Die der Zufuhr des Rückflusses dienende Leitung 18 zweigt von der Leitung 20 ab, durch die das Kopfprodukt aus der Abtriebskolonne 9 abgezogen wird, das folgende Zusammensetzung aufweist:

| | |
|---|---|
| Benzol | > 99,9 Gew.-% |
| Nichtaromaten | < 0,1 Gew.-% |
| Lösungsmittel | < 1 ppm |

In der Leitung 20 können gegebenenfalls noch in der Abbildung nicht dargestellte Kühleinrichtungen (Durchflußkühler) zur weiteren Abkühlung des durch die Leitung 20 fließenden Kohlenwasserstoffstromes vorgesehen sein.

Das Sumpfprodukt der Abtriebskolonne 9, welches im wesentlichen aus Lösungsmittel besteht, wird währenddessen über die Leitung 3 aus der Abtriebskolonne 9 abgezogen. Bei dem in der Abbildung wiedergegebenen Fließ-Schema passiert das Lösungsmittel in der Leitung 3 zunächst den Kocher 7, wo es in der weiter oben beschriebenen Art und Weise zur Beheizung der Extraktivdestillationskolonne 2 beiträgt. Im weiteren Verlauf der Leitung 3 sind dann im vorliegenden Falle die Wärmetauscher 21 und 22 vorgesehen, in denen ein Teil des Wärmeinhaltes des Lösungsmittels im indirekten Wärmeaustausch zur Lösungsmittelrückgewinnung aus der Raffinatphase (21) und Vorwärmung des Einsatzproduktes (22) herangezogen wird. Im Anschluß daran passiert das Lösungsmittel in der Leitung 3 schließlich den Kocher 13, in dem sein Wärmeinhalt in der weiter oben beschriebenen Art und Weise erfindungsgemäß genutzt wird. Während das Lösungsmittel bei Austritt aus der Abtriebskolonne 9 eine Temperatur von ca. 200°C aufweist, beträgt seine Temperatur hinter dem Umlaufkocher 13 nur noch ca. 100 – 103°C. Mit dieser Temperatur kann es ohne weitere Zwischenkühlung wieder auf die Extraktivdestillationskolonne 2 aufgegeben werden.

Es muß jedoch darauf hingewiesen werden, daß der Kocher 7 sowie die Wärmetauscher 21 und 22 nicht Elemente des erfindungsgemäßen Verfahrens sind. Diese sind lediglich im Falle des vorliegenden Verfahrensbeispieles angebracht. Wenn dagegen andere Einsatzprodukte und/ oder andere Lösungsmittel zur Anwendung gelangen und/oder in der Abtriebskolonne andere Temperaturverhältnisse bestehen, so können diese Einrichtungen gegebenenfalls ganz oder teilweise in Fortfall kommen, so daß es beispielsweise durchaus möglich ist, daß das aus dem Sumpf der Abtriebskolonne 9 abfließende Lösungsmittel unmittelbar in den Kocher 13 gelangt. Da dieser jedoch für die Beheizung der Abtriebskolonne 9 nicht ausreicht, ist zusätzlich am Kolonnensumpf noch in an sich bekannter Weise der Sumpfkocher 23 vorgesehen, der über die Leitung 24 mit Mitteldruckdampf beheizt wird. Das aufzuheizende Sumpfprodukt wird dabei über die Leitung 25 durch den Sumpfkocher 23 geführt und anschließend oberhalb der Entnahmestelle wieder in die Abtriebskolonne 9 eingeleitet. Durch die Anwendung des erfindungsgemäßen Verfahrens wird jedoch der Sumpfkocher 23 ganz erheblich entlastet, so daß dessen Mitteldruckdampfbedarf um ca. 35% gesenkt werden kann.

**Patentansprüche**

1. Verfahren zur Aufarbeitung des Sumpfproduktes von Extraktivdestillationsprozessen zur Gewinnung reiner Kohlenwasserstoffe, bei dem das aufzuarbeitende Sumpfprodukt der Extraktivdestillationskolonne in den mittleren Teil einer mit Böden und einem Sumpfkocher versehenen Abtriebskolonne eingeleitet wird, in der die zu gewinnenden Kohlenwasserstoffe über Kopf abdestilliert werden, während das Lösungsmittel aus dem Sumpf der Abtriebskolonne abgezogen und gegebenenfalls nach dem indirekten Wärmeaustausch mit anderen Produktströmen zur Lösungsmittelaufgabe der Extraktivdestillationskolonne zurückgeführt wird, dadurch gekennzeichnet, daß aus der Abtriebskolonne oberhalb des Aufgabebodens für das Sumpfprodukt der Extraktivdestillationskolonne über einen sogen. Rückflußverdampfungsboden mit erhöhtem Flüssigkeitsspiegel ein Seitenstrom abgezogen wird, der im indirekten Wärmeaustausch mit dem zur Extraktivdestillationskolonne zurückflie-ßenden Lösungsmittel aufgeheizt und danach in die Abtriebskolonne auf den Rückflußverdampfungsboden oder einen der darüberliegenden Böden wieder eingeleitet wird, wobei die Lösungsmittelkonzentration auf dem Rückflußverdampfungsboden durch die Rückflußmenge gesteuert und auf einem Wert zwischen 10 und 70 Gew.-% gehalten wird.

2. Verfahren nach Anspruch 1, dadurch gekennzeichnet, daß die Lösungsmittelkonzentration auf dem Rückflußverdampfungsboden auf einem Wert zwischen 20 und 50 Gew.-% gehalten wird.

3. Verfahren nach den Ansprüchen 1 und 2, dadurch gekennzeichnet, daß bei der Aufheizung des aus der Abtriebskolonne abgezogenen Seitenstromes das Lösungsmittel bis auf die Temperatur abgekühlt wird, mit der es wieder auf die Extraktivdestillationskolonne aufgegeben wird.

4. Verfahren nach den Ansprüchen 1 bis 3, dadurch gekennzeichnet, daß die Lösungsmittelkonzentration auf dem Rückflußverdampfungsboden gaschromatographisch oder durch Messung der Dichte oder des Berechnungsindexes oder der Temperatur bestimmt wird und der erhaltene Meßwert als Regelungsgröße für die Rückflußmenge dient.

5. Verfahren nach den Ansprüchen 1 bis 3, dadurch gekennzeichnet, daß die Temperaturdifferenz zwischen dem Rückflußverdampfungsboden und einem darüberliegenden Boden, vorzugsweise dem 4. bis 6. Boden oberhalb des Rückflußverdampfungsbodens, ermittelt wird und der erhaltene Δt-Wert als Regelungsgröße für die Rückflußmenge dient.

6. Verfahren nach Anspruch 5, dadurch gekennzeichnet, daß bei steigendem Δt-Wert die Rückflußmenge vergrößert und bei fallendem Δt-Wert verkleinert wird.

7. Verfahren nach den Ansprüchen 1 bis 6, dadurch gekennzeichnet, daß die Abtriebskolonne bei Normaldruck oder bei vermindertem Druck bis zu 0,15 bar betrieben wird.

## Claims

1. Process for working up the bottom product from extractive distillation processes, for the isolation of pure hydrocarbons, in which the bottom product, which is to be worked up, of the extractive distillation column is introduced into the middle section of a stripping column which is provided with trays and a reboiler and in which the hydrocarbons to be isolated are distilled off over the top, whilst the solvent is taken off at the bottom of the stripping column and, if appropriate after indirect heat exchange with other product streams, is recycled to the solvent feed of the extractive distillation column, characterised in that a sidestream is taken from the stripping column, above the feed tray for the bottom product from the extractive distillation column, via a so-called reflux-vaporising tray with a raised liquid level, which sidestream is heated up by indirect heat exchange with the solvent flowing back to the extractive distillation column and is then reintroduced into the stripping column onto the reflux-vaporising tray or one of the higher trays, the solvent concentration on the reflux-vaporising tray being controlled by the reflux rate and being maintained at a value between 10 and 70% by weight.

2. Process according to Claim 1, characterised in that the solvent concentration on the reflux-vaporising tray is preferably maintained at a value between 20 and 50% by weight.

3. Process according to Claims 1 and 2, characterised in that, when the sidestream taken from the stripping column is heated up, the solvent is preferably cooled down to the temperature at which it is recharged to the extractive distillation column.

4. Process according to Claims 1 to 3, characterised in that the solvent concentration on the reflux-vaporising tray is determined by gas chromatography or by measuring the density or calculation (sic) index or temperature, and the measured value obtained is used as the control parameter for the reflux rate.

5. Process according to Claims 1 to 3, characterised in that the temperature difference between the reflux-vaporising tray and a higher tray, preferably the 4th to 6th tray above the reflux-vaporising tray, is determined and the Δt value obtained is used as the control parameter for the reflux rate.

6. Process according to Claim 5, characterised in that the reflux rate is increased with a rising Δt value and is reduced with a falling Δt value.

7. Process according to Claims 1 to 6, characterised in that the stripping column is operated under normal pressure or under a reduced pressure of down to 0.15 bar.

## Revendications

1. Procédé de traitement du produit de queue de processus de distillation extractive pour l'obtention d'hydrocarbures purs, dans lequel on introduit le produit de queue de la colonne de distillation extractive, qu'il s'agit de traiter, dans la partie centrale d'une colonne d'épuisement munie de plateaux et d'un bouilleur de fond et dans laquelle on chasse par distillation à la tête les hydrocarbures à obtenir tandis que l'on retire le solvant du fond de la colonne d'épuisement et que, éventuellement après l'échange indirect de chaleur avec d'autres courants de produit, on le ramène à l'amenée de solvant de la colonne de distillation extractive, caractérisé par le fait que de la colonne d'épuisement, au-dessus du plateau d'amenée du produit de queue de la colonne de distillation extractive, on retire, en passant par un plateau dit d'évaporation au reflux à niveau de liquide plus élevé, un courant latéral que l'on chauffe en échange indirect de chaleur avec le solvant qui reflue à la colonne de distillation extractive et qu'ensuite on l'introduit à nouveau dans la colonne d'épuisement, sur le plateau d'évaporation au reflux ou sur l'un des plateau situés au-dessus, la concentration de solvant sur le plateau d'évaporation au reflux étant commandée par la quantité de reflux et étant maintenue à une valeur comprise entre 10 et 70% en poids.

2. Procédé selon la revendication 1, caractérisé par le fait que l'on maintient la concentration de solvant sur le plateau d'évaporation au reflux à une valeur comprise entre 20 et 50% en poids.

3. Procédé selon les revendications 1 et 2, caractérisé par le fait que lors du chauffage du courant latéral retiré de la colonne d'épuisement, on refroidit le solvant jusqu'à la température à laquelle il est à nouveau amené sur la colonne de distillation extractive.

4. Procédé selon les revendications 1 à 3, caractérisé par le fait que l'on détermine la concentration de solvant sur le plateau d'évaporation au reflux par chromatographie gazeuse ou en mesurant la densité ou l'indice de calcul ou la température et que la valeur mesurée obtenue sert de grandeur de réglage de la quantité de reflux.

5. Procédé selon les revendications 1 à 3, caractérisé par le fait que l'on détermine la différence de température entre le plateau d'évaporation au reflux et un plateau situé au-dessus, de préférence le 4ème à 6ème plateau au-dessus du plateau d'évaporation au reflux, et que la valeur $\Delta t$ obtenue sert de grandeur de réglage de la quantité de reflux.

6. Procédé selon la revendication 5, caractérisé par le fait que, lorsque la valeur $\Delta t$ augmente, on augmente la quantité de reflux et que, lorsque la valeur $\Delta t$ diminue, on diminue la quantité de reflux.

7. Procédé selon les revendications 1 à 6, caractérisé par le fait que l'on fait fonctionner la colonne d'épuisement à la pression normale ou à une pression réduite jusqu'à 0,15 bar.

7

0 046 876